Europäisches Patentamt

European Patent Office

Office européen des brevets

(19)

(11) Publication number: **0 594 224 A2**

(12) **EUROPEAN PATENT APPLICATION**

(21) Application number: **93120948.0**

(22) Date of filing: **15.09.86**

(51) Int. Cl.5: **C07H 19/073, A61K 31/70**

This application was filed on 27 - 12 - 1993 as a divisional application to the application mentioned under INID code 60.

(30) Priority: **17.09.85 US 776899**
**27.09.85 GB 8523878**
**12.02.86 GB 8603447**
**14.02.86 GB 8603719**
**04.04.86 GB 8608272**
**23.06.86 GB 8615322**
**23.06.86 US 877796**
**23.06.86 US 877284**

(43) Date of publication of application:
**27.04.94 Bulletin 94/17**

(60) Publication number of the earlier application in accordance with Art.76 EPC: **0 217 580**

(84) Designated Contracting States:
**AT BE CH DE FR GB IT LI LU NL SE**

(71) Applicant: **THE WELLCOME FOUNDATION LIMITED**
**Unicorn House**
**160 Euston Road**
**London NW1 2BP(GB)**

(72) Inventor: **Rideout, Janet Litster**
**3101 Morningside Drive**
**Raleigh, North Carolina 27607(US)**
Inventor: **Barry, David Walter**
**1810, South Lakeshore Drive**
**Chapel Hill, North Carolina 27514(US)**
Inventor: **Lehrman, Sandra Nusinoff**
**2720 Old Sugar Road**
**Durham, North Carolina 27707(US)**
Inventor: **St. Clair, Martha Heider**
**312 Seven Oaks Road**
**Durham, North Carolina 27704(US)**
Inventor: **Furman, Phillip Allen**
**910 Bluestone Road**
**Durham, North Carolina 27713(US)**
Inventor: **Freeman, George Andrew**
**107 Kerrwood Lane**
**Cary, North Carolina 27511(US)**
Inventor: **Zimmerman, Thomas Paul**
**1415, Brunson Court**
**Cary, North Carolina 27511(US)**
Inventor: **Wolberg, Gerlad**
**1109 Troon Court**
**Cary, North Carolina 27511(US)**
Inventor: **de Miranda, Paulo M.**
**4828 Radcliff Road**
**Raleigh, North Carolina 27609(US)**
Inventor: **Shaver, Sammy Ray**
**103 Autumn Drive**
**Chapel Hill, North Carolina 27514(US)**
Inventor: **White, Geoffrey**
**Coopers Animal Health Limited,**
**Berkhamsted Hill**
**Berkhamsted, Hertfordshire(GB)**
Inventor: **Kirk III, Leone Edward**
**1630 Sunrise Avenue**
**Raleigh, North Carolina 27608(US)**
Inventor: **King, Dannie Hilleary**
**8237 Hillside Drive**
**Raleigh, North Carolina 27612(US)**
Inventor: **Clemons, Richard Harlon**
**221 E Maynard Road**
**Cary, North Carolina 27511(US)**

(74) Representative: **Garrett, Michael et al**
**The Wellcome Foundation Limited**
**Group Patents and Agreements**
**Langley Court**
**Beckenham Kent BR3 3BS (GB)**

(54) **Therapeutic nucleosides.**

(57) 3'-Azido-3'-deoxythymidine or a pharmaceutically acceptable derivative thereof for the treatment or prophylaxis of Kaposi's sarcoma, multiple sclerosis or thrombocytopaenia purpura

The present invention relates to a 3'-azido-nucleoside and pharmaceutically acceptable derivatives thereof and their use in therapy for the treatment or prophylaxis of certain indications.

In the field of antiviral chemotherapy, few drugs exist which effectively control the virus per se, owing to the difficulty of attacking the virus while leaving uninfected host cells unimpaired. It was long supposed that, with the extreme parasitic nature of viruses, all the necessary facilities for viral replication were provided by the host cell. It has recently been established that certain stages in the virus life-cycle, which vary from species to species, are specified by the virus itself, and these stages may prove susceptible to attack where they differ sufficiently from any corresponding host-cell function. However, owing to great similarity between viral and host functions, effective treatments have proven very difficult to identity.

For this reason, compounds identified as being suitable for the treatment of viral infections usually have some toxicity for the host. Thus, the ideal medication is non-toxic at antivirally effective concentrations but, In the absence of such a treatment, the compound should possess a good therapeutic ratio, that is, the concentrations at which the treatment is toxic are significantly higher than those at which an antiviral activity is observed.

One group of viruses which has recently assumed a particular importance are the retroviruses. Retroviruses form a sub-group of RNA viruses which, in order to replicate, must first 'reverse transcribe' the RNA of their genome into DNA ('transcription' conventionally describes the synthesis of RNA from DNA). Once in the form of DNA, the viral genome may be incorporated into the host cell genome, allowing it to take full advantage of the host cell's transcription/translation machinery for the purposes of replication. Once incorporated, the viral DNA is virtually indistinguishable from the host's DNA and, in this state, the virus may persist for as long as the cell lives. As it is virtually invulnerable to attack in this form, any treatment must be directed at another stage of the virus life-cycle and will, of necessity, have to be continued until all virus-infected cells have died.

HTLV-I and HTLV-II are both retroviruses and are known to be causative agents of leukaemia in man. HTLV-I infections are especially widespread and are responsible for many deaths world-wide each year.

A species of retrovirus has also been reproducibly isolated from patients with AIDS. While it has been extensively characterised, there is still some dispute as to an internationally agreeable name for the virus. It is currently known either as human T-cell lymphotropic virus III (HTLV III), AIDS associated retrovirus (ARV) or lymphadenopathy associated virus (LAV) but it is anticipated that the name to be agreed on internationally will be human immunodeficiency virus (HIV). This virus (referred to herein as HIV) has been shown preferentially to infect and destroy T-cells bearing the OKT4 surface marker and is now generally accepted as the aetiologic agent of AIDS. The patient progressively loses this set of T-cells, upsetting the overall balance of the immune system, reducing his ability to combat other infections, and predisposing him to opportunistic infections which frequently prove fatal. Thus, the usual cause of death in AIDS victims is by opportunisitc infection, such as pneumonia or cancers which may be virally induced, and not necessarily as a direct result of HIV Infection. Other conditions associated with HIV infection include thrombocytopaenia purpura and Kaposi's sarcoma.

Recently, HIV has also been recovered from other tissue types, including B-cells expressing the T4 marker, macrophages and non-blood associated tissue in the central nervous system. This infection of the central nervous system is not necessarily associated with classical AIDS and has been found in patients with asymptomatic HIV infections. HIV infection of the CNS is associated with progressive demyelination, leading to wasting and such symptoms as encephalopathy, progressive dysarthria, ataxia and disorientation. Further conditions associated with HIV infection are the asymptomatic carrier state, progressive generalised lymphadenopathy (PGL) and AIDS-related complex (ARC).

It is now considered that certain, chronic, neurological infections are caused by retroviruses. Such infections include multiple sclerosis in man, for example, and caprine arthritis, encephalitis virus infections in goats, and visna-maedi infections in sheep.

Reports have described the testing of compounds against various retroviruses, for example, Friend Leukaemia Virus (FLV), a murine retrovirus. For instance Krieg et al (Exp.Cell Res., 116, (1978) 21-29) found that 3'-azido-3'-deoxythymidine affected the release of C-type particles and increased the formation of A-type particles of a FLV complex in vitro experiments, and Ostertag et al (Proc.Nat.Acad.Sci. (1974) 71, 4980-85) stated that, on the basis of antiviral activity related to the above FLV complex and a lack of cellular toxicity, 3'-azido-3'-dideoxythymidine "might favourable replace bromodeoxyuridine for medical treatment of diseases caused by DNA viruses". However, De Clerq et al (Biochem.Pharm. (1980) 29, 1849-1851) established, six years later, that 3'-azido-3'-dideoxythymidine had no appreciable activity against any viruses used in their tests, including such DNA viruses as vaccinia, HSVI and varicella zoster virus (VZV).

According to one feature of the present invention we provide 3'-azido-3'-deoxythymidine or a pharmaceutically acceptable derivative thereof for use in the treatment or prophylaxis of multiple sclerosis, Kaposi's Sarcoma or thrombocytopaenia purpura.

By "a pharmaceutically acceptable derivative" is meant any pharmaceutically acceptable salt, ester or salt of such ester, or any other compound which upon administration to the recipient, is capable of providing (directly or indirectly) the parent 3'-azidonucleoside or a therapeutically effective metabolite or residue thereof. An example of a non-ester compound is the derivative wherein the 5'-C and 2-C-atoms are linked by an oxygen atom to form an anhydro group.

Preferred esters include carboxylic acid esters in which the non-carbonyl moiety of the ester grouping is selected from straight or branched chain alkyl, alkoxyalkyl (e.g. methoxymethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy); sulphonate esters such as alkyl- or aralkylsulphonyl (e.g. methanesulphonyl); and mono-, di- or triphosphate esters. Any reference to any of the above compounds also includes a reference to a pharmaceutically acceptable salt thereof.

With regard to the above-described esters, unless otherwise specified, any alkyl moiety present advantageously containins 1 to 18 carbon atoms, particularly 1 to 4 carbon atoms. Any aryl moiety present in such esters advantegously comprises a phenyl group.

Examples of pharmaceutically acceptable salts and pharmaceutically acceptable derivatives thereof include base salts, e.g. derived from an appropriate base, such as alkali metal (e.g. sodium), alkaline earth metal (e.g. magnesium) salts, ammonium and $NX^{+}_{4}$ (wherein X is $C_{1-4}$ alkyl) and mineral acid salts, such as the hydrochloride.

Specific examples of pharmaceutically acceptable derivatives of 3'-azido-3'-deoxythymidine that may be used in accordance with the present invention include the monosodium salt and the following 5' esters: monophosphate; disodium monophosphate; diphosphate; triphosphate; acetate; 3-methyl-butyrate; octanoate; palmitate; 3-chloro benzoate; benzoate; 4-methyl benzoate; hydrogen succinate; pivalate; and mesylate.

3'-Azido-3'-deoxythymidine and its pharmaceutically acceptable derivatives, also referred to herein as the active ingredient, may be administered for therapy by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature of the infection and the chosen active ingredient.

In general a suitable dose will be in the range of 3.0 to 120 mg per kilogram body weight of the recipient per day, preferably in the range of 6 to 90 mg per kilogram body weight per day, and most preferably in the range 15 to 60 mg per kilogram body weight per day. The desired dose is preferably presented as two, three, four, five, six or more sub-doses administered at appropriate intervals throughout the day. These sub-doses may be administered in unit dosage forms, for example, containing 10 to 1500 mg, preferably 20 to 1000 mg, and most preferably 50 to 700 mg of active ingredient per unit dosage form.

Experiments with 3'-azido-3'-deoxythymidine suggest that a dose should be administered to achieve peak plasma concentrations of the active compound of from about 1 to 75 $\mu$M, preferably about 2 to 50 $\mu$M, most preferably about 3 to about 30 $\mu$M. This may be achieved, for example, by the intravenous injection of a 0.1 to 5% solution of the active ingredient, optionally in saline, or orally administered as a bolus containing about 1 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

While it is possible for the active ingredient to be administerd alone it is preferable to present it as a pharmaceutical formulation. The formulations of the present invention comprise at least one active ingredient, as above defined, together with one or more acceptable carriers thereof and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of an active ingredient.

3'-Azido-3'-deoxythymidine and its pharmaceutically acceptable derivatives, may be prepared in conventional manner using techniques that are well known in the art, e.g. as described in: Synthetic Procedures in Nucleic Acid Chemistry (1, 321 (1968), T.A.Krenitsky et al), J.Med.Chem. (26, 981 (1983)); Nucleic Acid Chemistry, Improved and New Synthetic Processes, Methods and Techniques (Parts 1 and 2, Ed. L.D.Townsend, R.S.Tipson, (J.Wiley) 1978); J.R.Horwitz et al. (J.Org.Chem. 29, (July 1964) 2076-78); M.Imazawa et al, (J.Org.Chem, 43, (15) (1978) 3044-3048); K.A.Watanabe et al. (J.Org.Chem., 45, 3274 (1980)); and R.P.Glinsky et al. (J.Chem.Soc.Commun., 915, (1970)), which disclosures are herein incorporated by reference, or using techniques that are analogous to those described therein.

The following Examples are intended for illustration only and are not intended to limit the scope of the invention in any way. The term 'Active Ingredient' as used in the Examples means 3'-azido-3'-deoxy thymidine or a pharmaceutically acceptable derivative thereof

Example 1: Tablet Formulations

The following formulations A and B are prepared by wet granulation of the ingredients with a solution of povidone, followed by addition of magnesium stearate and compression.

Formulation A

|  | mg/tablet | mg/tablet |
|---|---|---|
| (a) Active ingredient | 250 | 250 |
| (b) Lactose B.P. | 210 | 26 |
| (c) Povidone B.P. | 15 | 9 |
| (d) Sodium Starch Glycollate | 20 | 12 |
| (e) Magnesium Stearate | 5 | 3 |
|  | 500 | 300 |

4

Formulation B

|  | mg/tablet | mg/tablet |
|---|---|---|
| (a) Active ingredient | 250 | 250 |
| (b) Lactose 150 | - | - |
| (c) Avicel PH 101 | 60 | 26 |
| (d) Povidone B.P. | 15 | 9 |
| (e) Sodium Starch Glycollate | 20 | 12 |
| (f) Magnesium Stearate | 5 | 3 |
|  | 500 | 300 |

Formulation C

|  | mg/tablet |
|---|---|
| Active ingredient | 100 |
| Lactose | 200 |
| Starch | 50 |
| Povidone | 5 |
| Magnesium stearate | 4 |
|  | 359 |

The following formulations, D and E, are prepared by direct compression of the admixed ingredients. The lactose used in formulation E is of the direct compression type (Dairy Crest - "Zeparox").

Formulation D

|  | mg/capsule |
|---|---|
| Active Ingredient | 250 |
| Pregelatinised Starch NF15 | 150 |
|  | 400 |

Formulation E

|  | mg/capsule |
|---|---|
| Active ingredient | 250 |
| Lactose | 150 |
| Avicel | 100 |
|  | 500 |

Formulation F (Controlled Release Formulation)

The formulation is prepared by wet granulation of the ingredients (below) with a solution of povidone followed by the addition of magnesium stearate and compression.

5

|                                                              | mg/tablet |
|--------------------------------------------------------------|-----------|
| (a) Active Ingredient                                        | 500       |
| (b) Hydroxypropylmethylcellulose (Methocel K4M Premium)      | 112       |
| (c) Lactose B.P.                                             | 53        |
| (d) Povidone B.P.C.                                          | 28        |
| (e) Magnesium Stearate                                       | 7         |
|                                                              | 700       |

Example 2: Capsule Formulations

Formulation A

A capsule formulation is prepared by admixing the ingredients of Formulation D in Example 1 above and filling into a two-part hard gelatin capsule. Formulation B (infra) is prepared in a similar manner.

Formulation B

|                              | mg/capsule |
|------------------------------|------------|
| (a) Active ingredient        | 250        |
| (b) Lactose B.P.             | 143        |
| (c) Sodium Starch Glycollate | 25         |
| (d) Magnesium Stearate       | 2          |
|                              | 420        |

Formulation C

|                        | mg/capsule |
|------------------------|------------|
| (a) Active ingredient  | 250        |
| (b) Macrogol 4000 BP   | 350        |
|                        | 600        |

Capsules are prepared by melting the Macrogol 4000 BP, dispersing the active ingredient in tbe melt and filling the melt into a two-part hard gelatin capsule.

Formulation D

|                    | mg/capsule |
|--------------------|------------|
| Active ingredient  | 250        |
| Lecithin           | 100        |
| Arachis Oil        | 100        |
|                    | 450        |

Capsules are prepared by dispersing the active ingredient in the lecithin and arachis oil and filling the dispersion into soil, elastic gelatin capsules.

Formulation E (Controlled Release Capsule)

The following controlled release capsule formulation is prepared by extruding ingredients a, b and c using an extruder, followed by spheronisation of the extrudate and drying. The dried pellets are then coated with release-controlling membrane (d) and filled into a two-piece, hard gelatin capsule.

|  | mg/capsule |
|---|---|
| (a) Active Ingredient | 250 |
| (b) Microcrystalline Cellulose | 125 |
| (c) Lactose B.P. | 125 |
| (d) Ethyl Cellulose | 13 |
|  | 513 |

Example 3: Injectable Formulation

Formulation A

| Active ingredient | 0.200g |
|---|---|
| Hydrochloric acid solution, 0.1M   q.s. to pH | 4.0 to 7.0 |
| Sodium hydroxide solution, 0.1M   q.s. to pH | 4.0 to 7.0 |
| Sterile water   q.s. to | 10ml |

The active ingredient is dissolved in most of the water (35º-40ºC) and the pH adjusted to between 4.0 and 7.0 with the hydrochloric acid or the sodium hydroxide as appropriate. The batch was then made up to volume with the water and filtered through a sterile micropore filter into a sterile 10ml amber glass vials (type 1) and sealed with sterile closures and overseals.

Formulation B

| Active ingredient | 0.125g |
|---|---|
| Sterile, pyrogen-free, pH 7 phosphate buffer,   q.s. to | 25ml |

Example 4: Intramuscular injection

|  | Weight (g) |
|---|---|
| Active Ingredient | 0.20 |
| Benzyl Alcohol | 0.10 |
| Glycofurol 75 | 1.45 |
| Water for Injection   q.s. to | 3.00ml |

The active ingredient is dissolved in the glycofurol. The benzyl alcohol is then added and dissolved, and water added to 3 ml. The mixture is then filtered through a sterile micropore filter and sealed in sterile 3 ml amber glass vials (type 1).

Example 5: Syrup

Formulation A

|  | Weight (g) |
|---|---|
| Active Ingredient | 0.2500 |
| Sorbitol Solution | 1.5000 |
| Glycerol | 2.0000 |
| Sodium Benzoate | 0.0050 |
| Flavour, Peach 17.42.3169 | 0.0125ml |
| Purified Water   q.s. to | 5.0000 ml |

The active ingredient is dissolved in a mixture of the glycerol and most of the purified water. An aqueous solution of the sodium benzoate is then added to the solution, following by addition of the sorbitol solution and finally the flavour. The volume is made up with purified water and mixed well.

When the active ingredient is poorly soluble, the following formulation (B) is used.

Formulation B

|  | Weight (g) |
|---|---|
| Active ingredient | 0.250 |
| Sorbitol Solution | 1.500 |
| Glycerol | 0.005 |
| Dispersible Cellulose | 0.005 |
| Sodium Benzoate | 0.010 ml |
| Flavour |  |
| Purified Water   to | 5.000 ml |

Mix the sorbitol solution, glycerol and part of the purified water. Dissolve the sodium benzoate in purified water and add the solution to the bulk. Add and disperse the dispersible cellulose and flavour. Add and disperse the active ingredient. Make up to volume with purified water.

Example 6: Suppository

|  | mg/suppository |
|---|---|
| Active Ingredient (62$\mu$m)* | 250 |
| Hard Fat, BP (Witepsol H15 - Dynamit Nobel | 1770 |
|  | 2020 |

* The active ingredient is used as a powder wherein at least 90% of the particles are of 63$\mu$m diameter or less.

One-fifth of the Witepsol H15 is melted in a steam-jacketed pan at 45°C maximum. The active ingredient is sifted through a 200$\mu$m sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250 lm stainless steel screen and, with continuous stirring, is allowed to cool to 40°C. At a temperature of 38°C to 40°C, 2.02g of the mixture is filled into suitable, 2ml plastic moulds. The suppositories are allowed to cool to room temperature.

Example 7: Pessaries

|  | mg/pessary |
|---|---|
| Active ingredient (63μm) | 250 |
| Anhydrous Dextrose | 380 |
| Potato Starch | 363 |
| Magnesium Stearate | 7 |
|  | 1000 |

The above ingredients are mixed directly and pessaries prepared by direct compression of the resulting mixture.

Anti-ITP Activity of 3'-Azido-3'-deoxythymidine (AZT)

A patient having a platelet count of 38,000mm$^{-3}$ was diagnosed as having thrombocytopaenia purpura (platelet count <100,000mm$^{-3}$) and was treated for 6 weeks with 5mg/kg of AZT intravenously every 6 hours during which time, his platelet count rose to 140,000mm$^{-3}$. Treatment was then changed to 5mg/kg/4 hours orally for 4 weeks, discontinued for 4 weeks, when a drop in platelet numbers, to 93,000mm$^{-3}$ after 2 weeks, and to 70,000mm$^{-3}$ after 4 weeks, was seen. Treatment was recommenced at 5mg/kg/4 hours orally for 5 weeks and the platelet count rose to 194,000mm$^{-3}$. A reduction to 2.5mg/kg/4 hours orally indefinitely resulted in a slight decrease in platelet count, but not to an extent diagnostic of ITP.

Treatment of Kaposis Sarcoma with 3'-Azido-3'-deoxythymidine (AZT)

In this study, 9 patients diagnosed as having Kaposi's sarcoma (KS) were treated with AZT and the following effects observed.
A complete cure was effected in one patient.
Three patients exhibited regression of lesions.
In 2 patients the KS remained stable
In 3 patients lesions progressed.

**Claims**

1. 3'-Azido-3'-deoxythymidine or pharmaceutically acceptable derivative thereof for use in the treatment or prophylaxis of Kaposi's Sarcoma, feline leukaemia, multiple sclerosis or thrombocytopaenia purpura.

2. Use of 3'-azido-3-deoxythymidine or a pharmaceutically acceptable derivative thereof in the manufacture of a medicament for the treatment or prophylaxis of any of the conditions described in Claim 1.